# EUROPEAN PATENT SPECIFICATION

(11) **EP 4 425 162 B1**
(45) Date of publication and mention of the grant of the patent: **29.04.2026**
(21) Application number: 22924150.0
(22) Date of filing: 19.12.2022
(51) Int. Cl.: A61B 5/145, G01N 27/327, C12M 1/34

(54) **SAMPLE MEASURING DEVICE, SAMPLE MEASURING METHOD, AND SAMPLE MEASURING PROGRAM**
PROBENMESSVORRICHTUNG, PROBENMESSVERFAHREN UND PROBENMESSPROGRAMM
DISPOSITIF DE MESURE D'ÉCHANTILLON, PROCÉDÉ DE MESURE D'ÉCHANTILLON ET PROGRAMME DE MESURE D'ÉCHANTILLON

(30) Priority: 27.01.2022 JP 2022010830
(43) Date of publication of application: 04.09.2024
(73) Proprietor: PHC Holdings Corporation, Tokyo 100-8403 (JP)
(72) Inventor: IKETANI, Seiitirou, Toon-shi, Ehime, 791-0395 (JP); FUJITA, Sumitaka, Toon-shi, Ehime, 791-0395 (JP); FUKUMOTO, Satoshi, Toon-shi, Ehime, 791-0395 (JP)
(74) Representative: Grünecker Patent- und Rechtsanwälte PartG mbB
(86) International application number: PCT/JP2022/046633
(87) International publication number: WO 2023/145300

(56) References cited:
- EP-A1- 2 030 561
- WO-A1-2006/132250
- WO-A1-2013/073074
- JP-A- 2001 066 275
- JP-A- 2001 066 275
- JP-A- 2001 174 436
- JP-A- 2001 174 436
- JP-A- 2011 013 072
- JP-A- 2011 099 717
- JP-A- 2016 197 109
- JP-A- 2021 101 643
- JP-A- 2021 185 850
- JP-A- H11 281 609

## Description

The present invention relates to a sample measurement device, sample measurement method, and sample measurement program for measuring a sample during cell culture, for example.

A conventional cell culture analysis device comprises a base, a sensor that is fixed in a through-hole portion provided to the base, and a lead wire that is connected to the sensor for taking off signals.

For example, Patent Literature 1 discloses a sensor that is immersed in a liquid cell culture medium and measures the cell culture environment within the medium.

Patent Literature 2 discloses a configuration comprising a lifting mechanism (elevator) for disposing a sensor in a culture medium.

Patent Literature 3 discloses a device and a method with which a sensor is immersed in a medium in a container, and cells contained in the medium are analyzed.

EP 2 030 561 A1 relates to a measuring system with a three-part electrode system and a potentiostat that controls a voltage between a working electrode and a reference electrode such that there is no current flowing through the reference electrode. The potentiostat measures the current between the electrodes and generates a voltage used for calculating a concentration of an analyte. The potential of a counter electrode is monitored and used for detection of malfunctions, if said voltage is outside of a certain reference range.

An ion concentration measuring device configured to detect contamination of the electrodes is also known from JP 2001 174436 A.
Patent Literature 1: U.S. Unexamined Patent Application Publication No. 2014/0186876
Patent Literature 2: U.S. Unexamined Patent Application Publication No. 2016/0077083
Patent Literature 3: U.S. Patent No. 7,638,321

However, the conventional sensors, devices, and methods mentioned above have the following problem.

Cell culture is generally carried out by pouring a medium into a well plate including a plurality of recessed portions (wells), so there is sometimes variance in the amount of medium put into each well. Consequently, in performing cell culture with the sensor immersed in the medium, there is the risk that the worker may forget to add the medium, or that the part of the sensor including the measurement electrode may not be sufficiently immersed due to variance in the amount of medium, resulting in measurement error.

Also, since cell culture is performed in an environment of high-temperature and - humidity, there is the risk that mold or other such impurities (contamination) may adhere to the sensor and adversely affect the measurement result.

Accordingly, the conventional configuration described above did not take into account the possibility of improper sensor immersion or contamination by mold or the like, making it difficult to accurately detect the occurrence of measurement errors attributable to improper immersion of the sensor, contamination, or the like.

It is an object of the present invention to provide a sample measurement device according to independent claim 1, sample measurement method according to independent claim 12, and sample measurement program according to independent claim 13 with which measurement errors attributable to contamination can be detected in order to make accurate measurements.

The invention is defined by said independent claims. The dependent claims 2 to 11 describe advantageous embodiments.

With the sample measurement device according to the present invention, the occurrence of measurement errors attributable to contamination can be detected in order to perform more accurate measurement.

### BRIEF DESCRIPTION OF THE DRAWINGS

Fig. 1 is an overall view of a cell culture analysis device comprising the sensor unit according to an embodiment of the present invention;
Fig. 2 is an oblique view of the configuration of an analysis unit included in the cell culture analysis device in Fig. 1;
Fig. 3 is an oblique view of a state in which a culture module equipped with a sensor is placed in the analysis unit of Fig. 2;
Fig. 4 is a schematic diagram showing processing on the sensor-equipped culture module in a biological safety cabinet in a room temperature environment;
Fig. 5 is a top view showing a well plate including a plurality of wells in which the lower ends of the sensors included in the sensor-equipped culture module of Fig. 4 are immersed;
Fig. 6 is an oblique view of the plurality of sensors included in the sensor-equipped culture module in Fig. 4;
Fig. 7 is an oblique view of a state in which the lower end of a sensor in Fig. 6 is immersed in a medium in a well;
Fig. 8 is plan view of the configuration of the sensor shown in Fig. 6;
Fig. 9A is a cross-sectional view of the configuration of a working electrode unit included in the electrode unit of the sensor in Fig. 8, and Fig. 9B is a cross-sectional view of the configuration of a counter electrode and a reference electrode included in the electrode unit of the sensor in Fig. 8;
Fig. 10 is a control block diagram of the cell culture analysis device in Fig. 1;
Fig. 11 is a diagram illustrating the behavior of the counter electrode in the circuit configuration of the measurement unit of the sensor shown in Fig. 8;
Fig. 12A is a graph of the relation between elapsed time and the sensor current value, and Fig. 12B is a graph of the relation between elapsed time and the terminal voltage of the counter electrode;
Figs. 13A, 13B, and 13C are graphs of the relation between elapsed time and the counter electrode terminal voltage under normal conditions, when contamination has occurred, and when the immersion is improper;
Fig. 14 is a flowchart showing the processing flow of a sample measurement method that makes use of the cell culture analysis device in Fig. 1;

### DESCRIPTION OF THE EMBODIMENTS

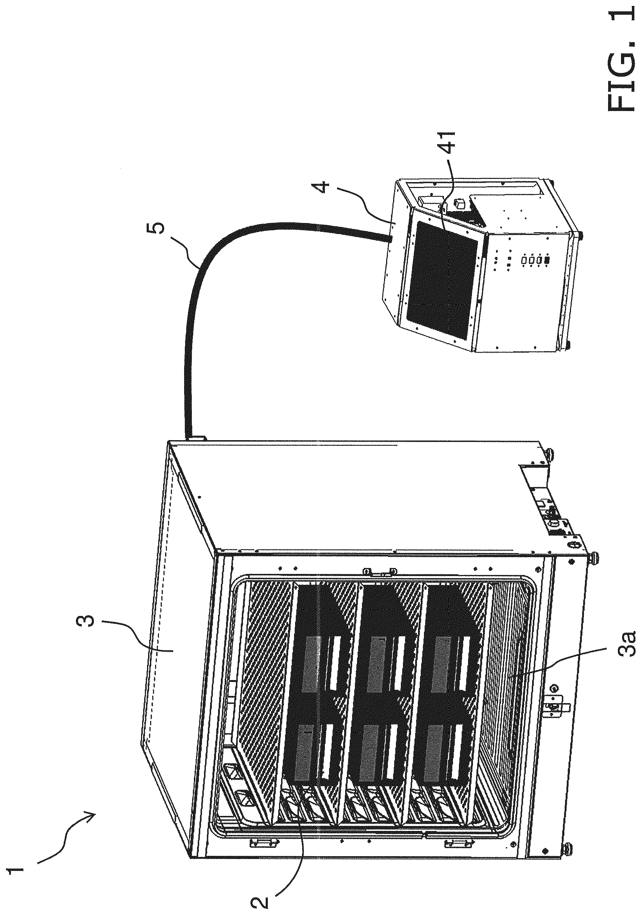

A cell culture measurement device (sample measurement device) 1 according to an embodiment of the present invention will now be described through reference to Figs. 1 to 14.

In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the patent claims.

### Overview of Cell Culture Analysis Device 1

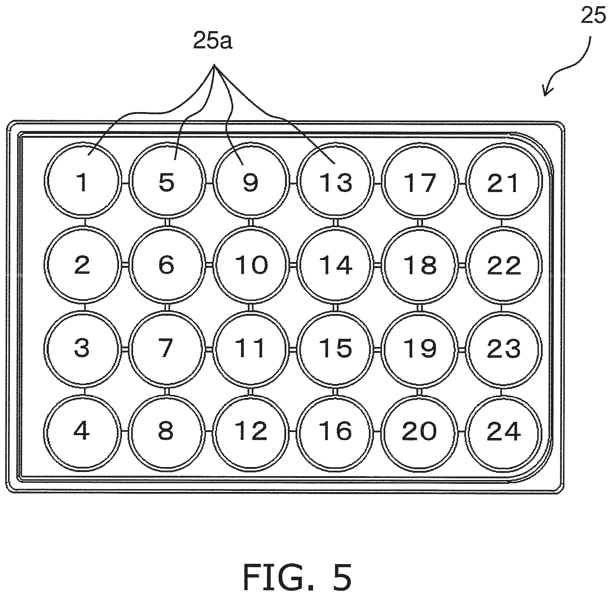

The cell culture analysis device 1 of this embodiment electrochemically senses the concentration of a specific component (such as glucose or lactic acid) contained in a medium X in a state in which part of a sensor (sample measurement sensor) 30 has been immersed in a culture medium (liquid sample) held in a well plate 25 (see Fig. 5) including a plurality of wells (culture vessels), and analyzes the culture state (cell metabolism, etc.).

As shown in Fig. 1, the cell culture analysis device 1 comprises an analysis unit 2, an incubator 3 in the interior space of which the analysis unit 2 is placed, and a control unit 4 that controls the analysis unit 2 and displays analysis results. Also, the analysis unit 2 and the control unit 4 are connected by an electrical cable 5.

A transparent door 3a attached to the front of the incubator 3 is opened, and the analysis unit 2 is placed in the internal space. The control unit 4 connected to the analysis unit 2 via the electrical cable 5 is disposed on the outside of the incubator 3.

This allows the user to use the control unit 4 to analyze the culture state in the incubator 3 without opening or closing the door 3a of the incubator 3, and thereby prevents contamination of the air in the incubator 3.

The analysis unit 2 is designed to be short in the lateral (width) direction, short in the height direction, and long in the depth direction so that a plurality of analysis units 2 can be installed in the incubator 3.

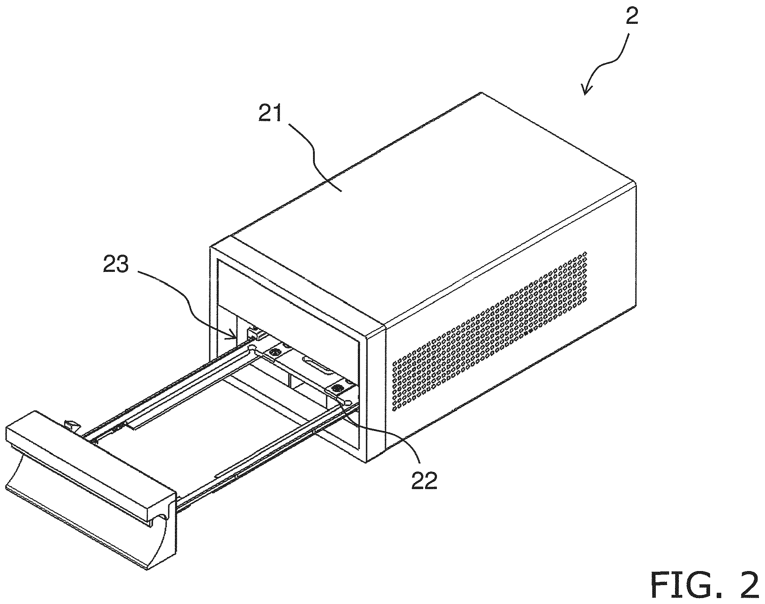

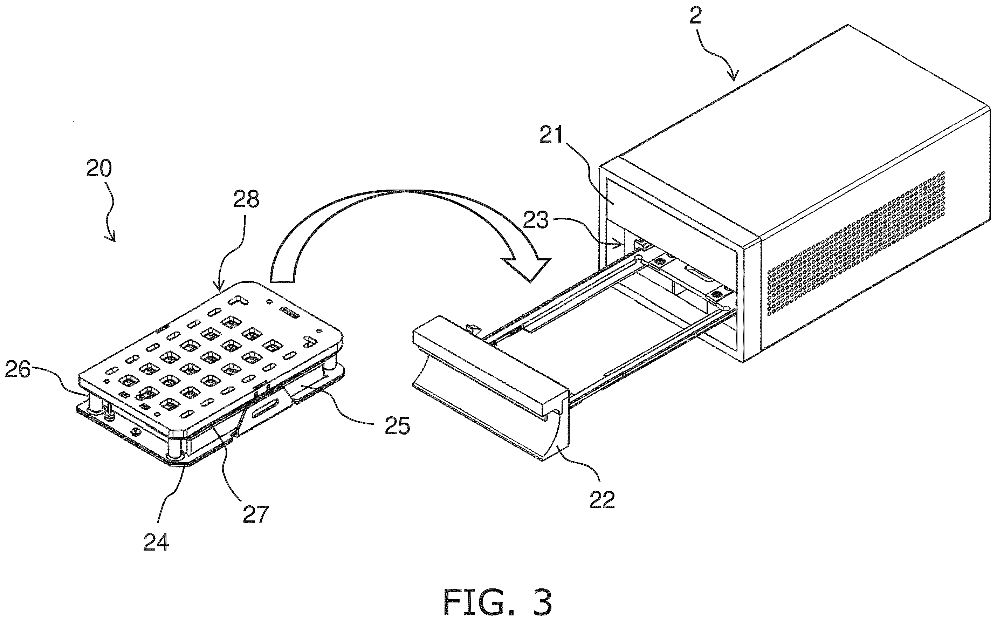

As shown in Figs. 2 and 3, the analysis unit 2 comprises a sensor-equipped culture module 20, a main body 21, a pull-out part 22, and a lifting mechanism 23.

Also, as shown in Fig. 1, the analysis unit 2 is disposed in the incubator 3 in advance, and is connected to the control unit 4 by the electrical cable 5. Then, during cell culture analysis, the assembled sensor-equipped culture module 20 is placed by the user in the main body 21 inside the incubator 3, as shown in Fig. 3.

The analysis unit 2 is configured to be lifted toward a probe holder (not shown) by an lifting mechanism 23 in a state in which the sensor-equipped culture module 20 has been pulled into the main body 21 by the pull-out part 22.

The detailed configuration of the analysis unit 2 will be described in detail below.

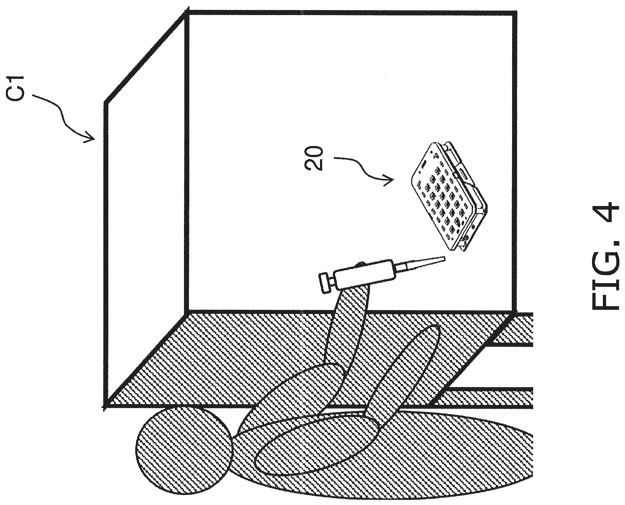

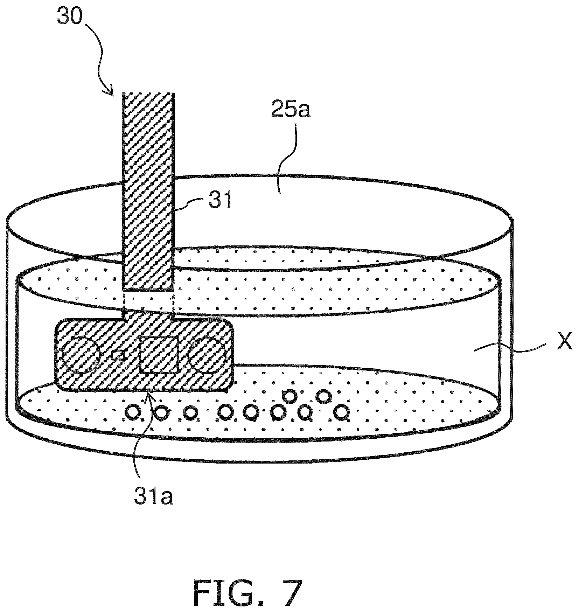

As shown in Fig. 4, the sensor-equipped culture module 20 is assembled after the wells 25a of the well plate 25 shown in Fig. 5 have been filled with the medium X (see Fig. 7) and cells have been seeded, inside a biological safety cabinet C1 under a room-temperature environment (such as an air temperature of 25 degrees). The assembled sensor-equipped culture module 20 is placed in the analysis unit 2 inside the incubator 3, which is maintained at a high-temperature and -humidity environment (37 degrees, humidity of 90% or higher). That is, the assembled sensor-equipped culture module 20 is moved from a room-temperature environment to a high-temperature and -humidity environment (such as a temperature of 37 degrees and a humidity of 90% or more).

The configuration for suppressing the occurrence of measurement errors, including contamination errors attributable to the adhesion of mold or other impurities to the sensor surface, and improper immersion errors in which the sensor 30 is not sufficiently immersed in the medium X, after the sensor-equipped culture module 20 is moved from a room-temperature environment to a high-temperature and -humidity environment, will be described in detail below.

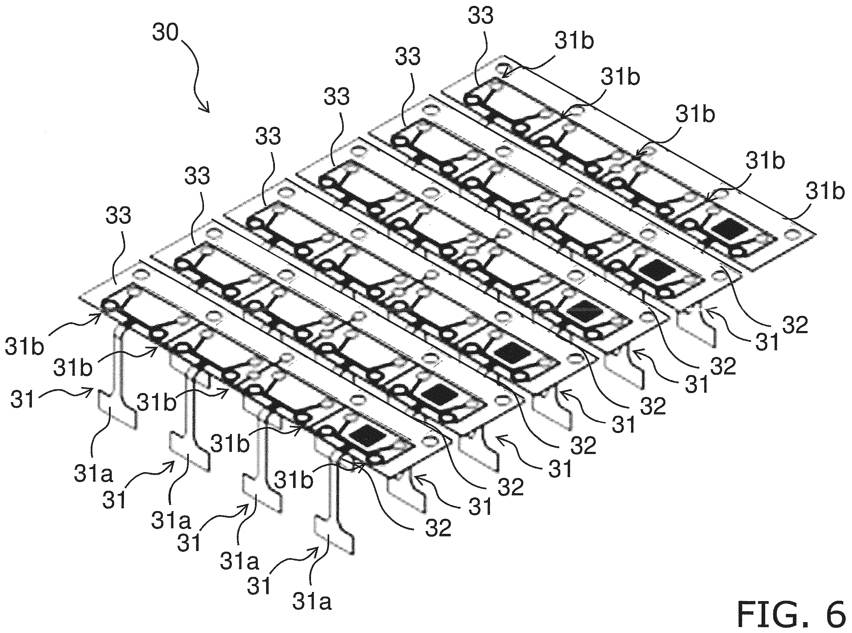

The sensor 30 is configured, for example, by forming a carbon electrode layer by sputtering on the upper surface of a PET (polyethylene terephthalate) film, which is a resin material. As shown in Fig. 6, the sensor 30 has a main body portion 31, a sensing unit (electrode unit) 31a, a connecting terminal portion 31b, a bent portion 32, and a linking portion 33.

The main body portion 31 is a substantially rectangular, flat member, and is linked at its upper end to the bent portion 32.

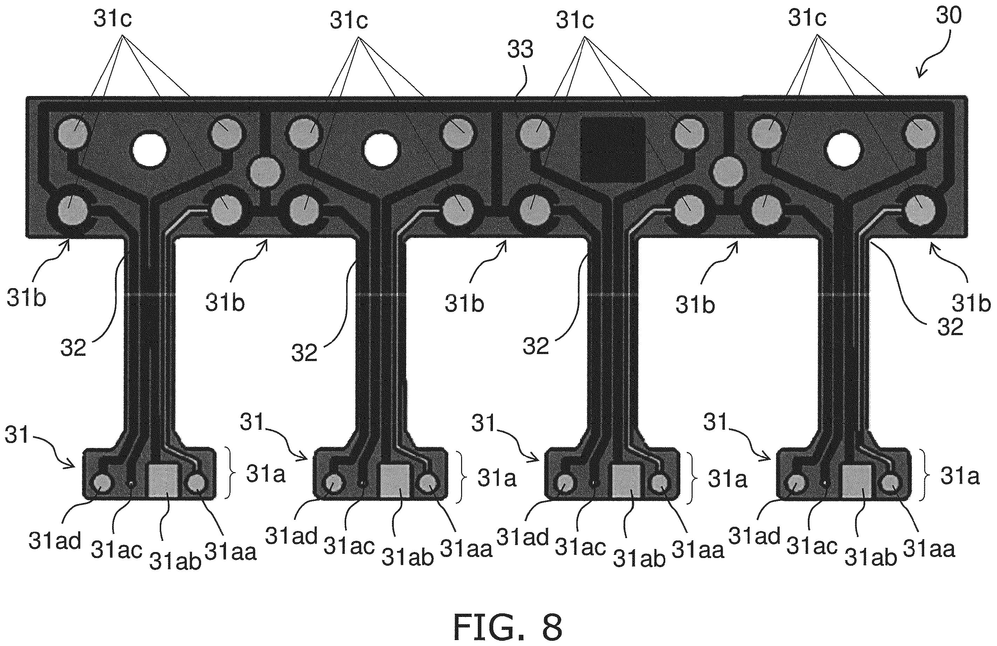

As shown in Fig. 7, the sensing unit (electrode unit) 31a is provided at the lower end of the main body portion 31, and a specific voltage is applied to each measurement electrode while immersed in the well 25a containing medium X, which allows the concentration of a specific component (such as glucose, or lactic acid) contained in medium X to be electrochemically measured. As shown in Fig. 8, the sensing unit 31a is provided on the surface of the wide part of the lower end of the downwardly facing T-shaped main body portion 31, and includes measurement electrodes (working electrodes 31aa and 31ad, counter electrode 31ab, reference electrode 31ac).

The measurement electrodes included in the sensing unit 31a are formed by transpiring the electrode layer with a laser and dividing the electrode layer. An electrode pattern may be formed by screen printing on each measurement electrode in order to improve the insulation between the wiring.

Here, when measuring the concentration of glucose contained in the medium X, for example, the reagent layer immobilized on the surface of the working electrodes may contain a glucose oxidase, such as glucose oxidase (GOx), glucose dehydrogenase (GDH), or even a redox mediator.

The concentration of glucose is measured by allowing the glucose that has permeated from the medium X through a protective film to react with the enzyme in the reagent layer (such as Gox or GDH) and be oxidized into gluconolactone, and converting the reduced product of a redox mediator produced at the same time, or the electrons generated by the oxidation reaction of hydrogen peroxide, into a current value.

As shown in Fig. 6, the bent portion 32 is a portion that links the main body portion 31 and the linking portion 33, and is bent approximately at a right angle along a specific bending line. Consequently, the linking portion 33 is disposed substantially perpendicular to the main body portion 31.

As shown in Figs. 6 and 8, the linking portion 33 links the upper ends of the main body portions 31 of the four sensors 30 disposed in the lateral direction to each other via the bent portions 32.

As shown in Fig. 8, the connection terminal portions 31b are connected to the four electrodes 3 1c disposed corresponding to the measurement electrodes of the sensing unit 31a of one sensor 30, which is a set of four.

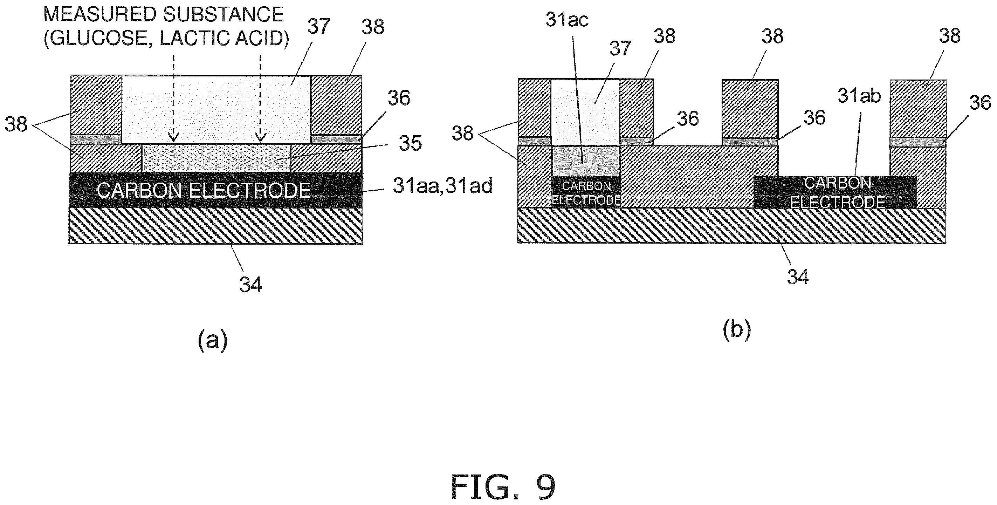

Of the four measurement electrodes included in the sensing unit 31a, the working electrode 31aa is disposed at the left end of the main body portion 31 of the sensor 30, and includes a reagent layer 35 coated with a reagent for measuring lactic acid. Also, as shown in Fig. 9A, the working electrode 31aa is provided as an electrode that is formed on the upper surface of a base material 34. The reagent layer 35 is provided on the upper surface of the portion of the working electrode 31aa that is used as an electrode. An insulating layer 38 is provided around the reagent layer 35 so as to surround the reagent layer 35. Furthermore, a protective film 37 that allows the object to be measured (lactic acid) to pass through is provided on the upper surface of the reagent layer 35. In addition, an insulating layer 38 is provided on the upper surface of the insulating layer 38 with an adhesive layer 36 interposed therebetween.

Consequently, since the reagent layer 35 is provided on the upper surface of the working electrode 31aa, the lactic acid that has permeated through the protective film 37 reacts with the reagent in the reagent layer 35, which causes the current value measured at the working electrode 31aa to change. This allows a change in the concentration of lactic acid contained in the medium X to be detected by detecting a change in this current value.

The counter electrode 31ab is disposed between the working electrode 31aa and the reference electrode 31ac in the main body portion 31 of the sensor 30. Also, as shown in Fig. 9B, the counter electrode 31ab is provided in an exposed state, without the reagent layer or the protective film being provided, so that its surface is in direct contact with the medium X.

Consequently, in measuring the counter electrode terminal voltage (discussed below), if there are impurities (contamination) such as mold that cover the surface of the counter electrode 31ab, for example, a contamination error can be detected very accurately by detecting with high sensitivity any change in the counter electrode voltage that occurs as a result of a reduction in the surface area of the counter electrode 31ab.

Also, the electrode portion of the counter electrode 31ab is formed from carbon. Since this carbon is more textured and porous than an electrode made of gold or the like, mold easily adheres to the surface of the counter electrode 31ab, making it more likely that the reaction surface area of the electrode will decrease and the occurrence of contamination will be detected.

As shown in Fig. 8, the reference electrode 31ac is disposed between the counter electrode 31ab and the working electrode 31ad in the main body portion 31 of the sensor 30. The reference electrode 31ac is formed from silver chloride, and its surface is covered with the protective film 37 as shown in Fig. 9B.

The working electrode 31ad is disposed at the right end of the main body portion 31 of the sensor 30, and includes the reagent layer 35 coated with a reagent for measuring glucose. Also, as shown in Fig. 9A, the working electrode 31ad is provided as an electrode that is formed on the upper surface of the base material 34, similarly to the working electrode 31aa. The reagent layer 35 is provided on the upper surface of the portion of the working electrode 31ad that is used as an electrode. The insulating layer 38 is provided around the reagent layer 35 so as to surround the reagent layer 35. Furthermore, the protective film 37 that allows the object to be measured (glucose) to pass through is provided on the upper surface of the reagent layer 35. Another insulating layer 38 is provided on the upper surface of the insulating layer 38 with an adhesive layer 36 interposed therebetween.

Consequently, since the reagent layer 35 is provided on the upper surface of the working electrode 31ad, the glucose that has permeated through the protective film 37 reacts with the reagent in the reagent layer 35, resulting in a change in the current value measured at the working electrode 31ad. This allows a change in the concentration of glucose contained in the medium X to be detected by detecting the change in this current value.

The protective film 37 is provided to prevent silver chloride or the like provided on the reagent layer 35 and the reference electrode 31ac containing reagents that are toxic to the cells being cultured from eluting into the medium X. Also, providing the protective film 37 makes possible long-term measurement, such as for 14 days, by suppressing the permeability of the analyte (glucose and lactic acid) and limiting the reactivity of the reagent contained in the reagent layer 35.

Here, the working electrodes 31aa and 31ad are formed so as to be exposed through an opening that is substantially circular in plan view. This allows the protective film 37 provided on the upper surfaces of the working electrodes 31aa and 31ad to be applied substantially evenly.

On the other hand, as discussed above, the counter electrode 31ab is not provided with a reagent layer or a protective film, so it is formed to be discharged from a substantially rectangular opening so that the surface area in contact with the medium X will be as large as possible. This improves the sensitivity at which the counter electrode terminal voltage is measured when a measurement error is detected (discussed below).

The four electrodes 31c are provided to the upper part of the sensor 30 as contacts that are electrically connected to the cell culture analysis device 1 (probe holder (not shown)). The electrodes 31c are electrically connected to the measurement electrodes (working electrodes 31aa, 31ad, counter electrode 31ab, reference electrode 31ac) included in the sensing unit 31a disposed at the lower part of the main body portion 31 of the sensor 30.

In this embodiment, as shown in Fig. 6, the plurality of sensors 30 included in the sensor unit 28 each comprise the main body portion 31, the sensing unit 31a that is disposed on the lower end side of the main body portion 31, is immersed in the medium X, and measures the components of the medium X, and the linking portion 33 that links the plurality of sensors 30 on the upper end side of the main body portion 31.

Consequently, since the sensors 30 are attached to the upper surface of the bottom plate 27 in a state of being linked together by the linking portions 33, the positions of the linked sensors 30 can be accurately defined.

This improves the positional accuracy (position, angle, etc.) of each sensor 30 with respect to the plurality of wells (culture vessels) 25a included in the well plate 25.

As a result, the immersion depth of each sensor 30 in the medium X placed in a well 25a becomes substantially constant, which allows stable measurement results to be obtained.

Meanwhile, although the positional accuracy of each sensor 30 with respect to the well 15a is improved, the well plate 25 includes a plurality of (24 in this embodiment) wells 25a. Accordingly, it is difficult to accurately pour the specified amount of medium X into each well 25a, and there is the risk that errors such as forgetting to add the medium X, or pouring in the wrong amount may occur. Consequently, there is the risk that there will be variance in the amount of medium X put into each well 25a, and when the sensing unit 31a of the sensor 30 is immersed in the medium X in the well 25a, that an improper immersion error may occur in which there is not enough medium X and the sensing unit 31a is not sufficiently immersed.

Furthermore, since the environment in which cell culture is performed is a high-temperature and -humidity environment as mentioned above, mold or other such impurities (contaminants) are more apt to be a problem during cell culture. For example, if mold or other such impurities (contaminants) adhere to and grow on the surface of the sensor 30 while the sensor is immersed in the medium X, there is the risk of adversely affecting the measurement result for the measurement target (glucose, lactic acid).

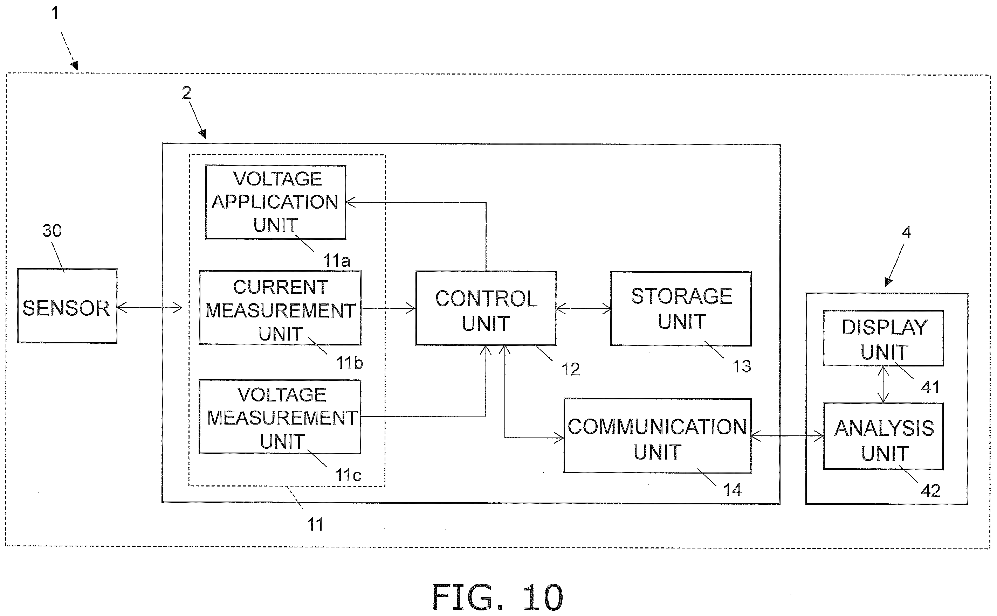

With the cell culture analysis device 1 of this embodiment, in order to detect the occurrence of such measurement errors (improper immersion error, contamination error) with high accuracy, the analysis unit 2 comprises an electrochemical measurement unit 11, a control unit 12, a storage unit 13, and a communication unit 14 as shown in Fig. 10.

The electrochemical measurement unit 11 is a potentiostat that measures the concentration of a measurement target by applying a specific voltage to the electrodes of a sensor 30 having a three-electrode configuration, and as shown in Fig. 10, has a voltage application unit 11a, a current measurement unit 11b, and a voltage measurement unit (counter electrode terminal voltage measurement unit) 11c.

The voltage application unit 11a applies a specific voltage for measuring the concentration of glucose and lactic acid contained in the medium X to the sensor 30 having the three-electrode configuration mentioned above.

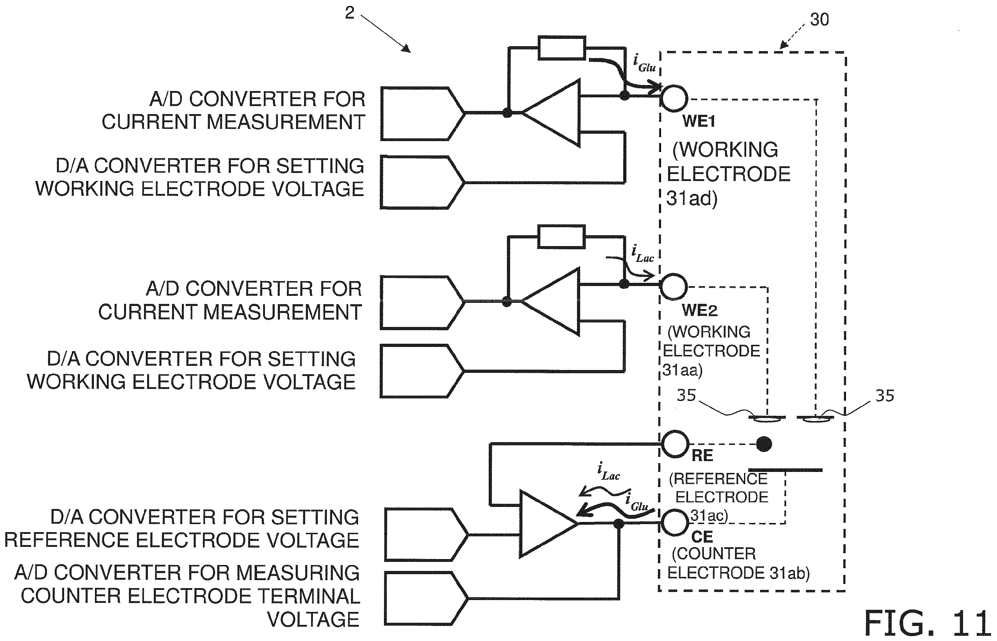

The current measurement unit 11b detects a change in the value of the current flowing through the sensor 30, which is measured by applying a voltage to the electrodes of the sensor 30 from the voltage application unit 11a. This allows the concentration of lactic acid to be measured according to the change in the value of the current flowing through the working electrode 31aa, and the concentration of glucose to be measured according to the change in the value of the current flowing through the working electrode 31ad (see Fig. 11).

The voltage measurement unit 11c measures the terminal voltage of the counter electrode 31ab of the sensor 30 in order to detect the occurrence of a measurement error (improper immersion error, contamination error). The result measured by the voltage measurement unit 11c is transmitted to the control unit 4 via the communication unit 14.

As shown in Fig. 10, the control unit 12 is connected to the voltage application unit 11a, the current measurement unit 11b, the voltage measurement unit 11c, the storage unit 13, and the communication unit 14. The control unit 12 controls the voltage application unit 11a so that a specific voltage will be applied to the sensor 30, and controls the communication unit 14 so that the measurement results from the current measurement unit 11b and the voltage measurement unit 11c will be transmitted to the control unit 4.

As shown in Fig. 10, the storage unit 13 is connected to the control unit 12, and stores data such as the preset value of the applied voltage for each measurement target, or the measurement results from the current measurement unit 11b and the voltage measurement unit 11c, for example.

As shown in Fig. 10, the communication unit 14 is controlled by the control unit 12 and transmits data such as the measurement results from the current measurement unit 11b and the voltage measurement unit 11c to an analysis unit 42 of the control unit 4.

As shown in Fig. 10, the control unit 4 is capable of communicating with the analysis unit 2 via the communication unit 14, and comprises a display unit 41 and an analysis unit 42.

The display unit 41 displays, as the result of analysis by the analysis unit 42, the change in the concentration of glucose and lactic acid based on the sensor current value measured by the current measurement unit 11b, the measurement error detection result based on the value of the counter electrode terminal voltage measured by the voltage measurement unit 11c, and so forth.

The analysis unit 42 is, for example, a PC (personal computer), and performs metabolic analysis of the cells during culture by measuring the concentrations of lactic acid and glucose (the measurement targets) on the basis of the change in the sensor current flowing through the working electrodes 31aa and 31ad as measured by the current measurement unit 11b. Also, the analysis unit 42 detects whether there is any measurement error (contamination error, improper immersion error; (discussed below)) on the basis of the change in the counter electrode terminal voltage measured by the voltage measurement unit 11c.

### Measurement Error Detection

The cell culture analysis device 1 of this embodiment has the configuration described above, and measures the counter electrode terminal voltage with the voltage measurement unit 11c to detect any measurement errors, including contamination errors in which mold or another such impurity adheres to the sensor surface and reduces the accuracy of the measurement result, and improper immersion errors in which the sensor is not sufficiently immersed due to variance in the amount of medium X put into the wells 25a of the well plate 25.

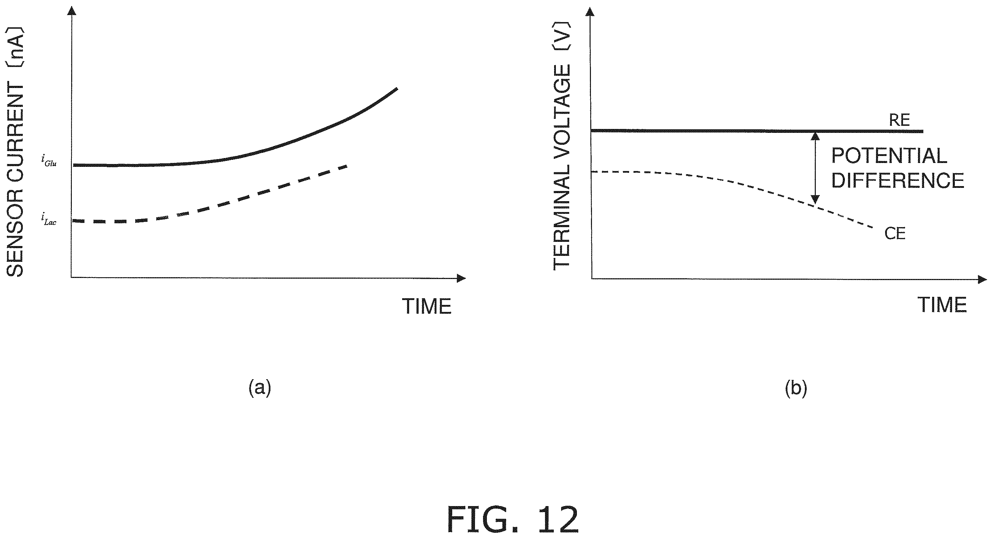

Here, the principle by which measurement errors can be detected by measuring the counter electrode terminal voltage will be explained through reference to Figs. 11, 12A, and 12B.

Figs. 12A and 12B are graphs intended to facility an understanding of the behavior of the counter electrode terminal voltage, and in actual measurement, cell metabolism consumes glucose and produces lactic acid, so the glucose current value decreases over time, while the lactate current value increases.

When the circuit within the sensor 30 shown in Fig. 11 is operating normally, the currents i_{Lac} and i_{Glc} flowing through the counter electrode 31ab and the working electrodes 31aa and 31ad are equivalent. Then, as shown in Fig. 12A, when the currents i_{Lac} and i_{Glc} flowing through the working electrodes 31aa and 31ad increase, this changes the counter electrode terminal voltage in the direction in which the current on the counter electrode 31ab side increases.

In other words, a change in the counter electrode terminal voltage in the direction in which the current on the counter electrode 31ab side increases is equivalent to an increase in the reaction per unit of electrode surface area in the counter electrode 31ab. This means that the drive force of the counter electrode 31ab is greater, that is, as shown in Fig. 12B, the voltage difference (RE - CE) between the reference electrode 31ac and the counter electrode 31ab is greater.

Therefore, in terms of the circuit, as shown in Fig. 12B, the operational amplifier output (counter electrode terminal voltage) connected to the counter electrode 31ab changes in the negative direction. That is, as the sensor current rises, the counter electrode terminal voltage does down.

Consequently, when impurities such as mold are generated and proliferate on the surface of the sensor 30 installed in a high-temperature and -humidity environment, and particularly on the surface of the counter electrode 31ab, the counter electrode terminal voltage drops off sharply, for example.

Because of this, with the cell culture analysis device 1 of this embodiment, by measuring the counter electrode terminal voltage, contamination errors and improper immersion errors occurring in the sensor 30 are detected, which avoids measurement of the concentration of glucose concentration and lactic acid from being made in a state of diminished accuracy.

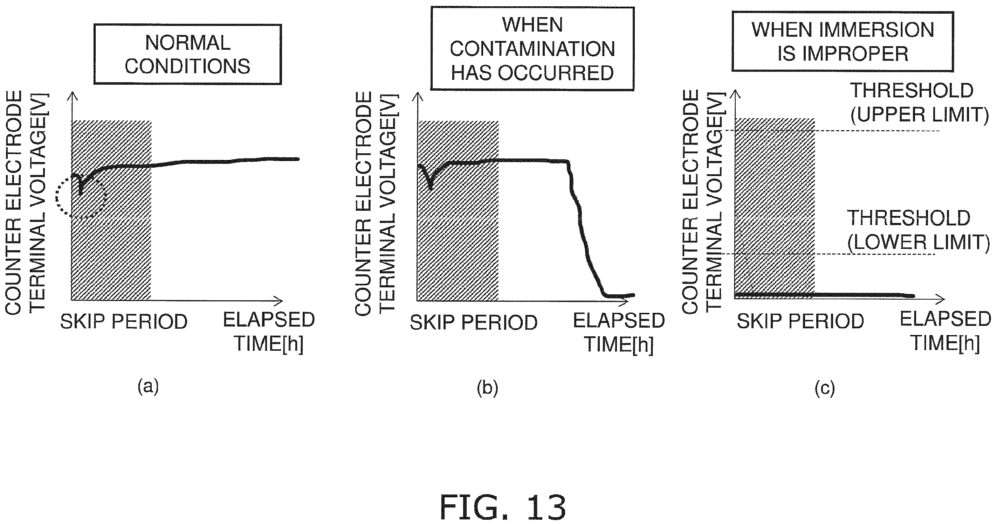

More specifically, with the cell culture analysis device 1 of this embodiment, as shown in Fig. 13A, the analysis unit 42 sets a skip period in which no measurement is performed for a specific length of time from the start of voltage application to the sensor 30.

This skip period is set because, for several hours immediately after the start of voltage application to the sensor 30, there is the risk that the counter electrode terminal voltage will rise change significantly depending on the state of the sensor 30 before voltage application, so there is the risk that there will be variance in the measurement result.

After the sensor 30 is immersed in the medium X, up until the point when the voltage is applied, the action of the mediator reagent causes the amount of reducing substances to increase. This creates a difference in the magnitude of the sensor current flowing immediately after voltage application depending on how long it takes from immersion of the sensor 30 until the application of voltage. That is, the longer is the time from immersion to voltage application, the larger is the amount of sensor current. Therefore, in terms of the circuit, in order to cause a large current equivalent to that of the working electrodes 31aa and 31ad to flow through the counter electrode 31ab, the terminal voltage of the counter electrode 31ab is controlled to decrease.

For this reason, the several hours immediately after the start of voltage application, in which the rate of fluctuation of the counter electrode terminal voltage is more likely to increase, is set as a skip period (dead zone).

The detection of a contamination error will now be described through reference to Fig. 13B.

As shown in Fig. 13B, when an impurity such as mold is generated and proliferates on the surface of the sensor 30 after the skip period has elapsed, the counter electrode terminal voltage drops off sharply midway through.

At this point, the counter electrode terminal voltage also decreases due to cell metabolism, but if there is contamination, this decrease will be even sharper than during cell metabolism (the negative rate of change is higher than during cell metabolism).

Consequently, the analysis unit 42 determines whether or not the negative rate of change in the counter electrode terminal voltage is below a specific threshold value, and if it is below the specific threshold value, it is determined that a contamination error has occurred, and the display unit 41 displays that a contamination error has occurred, which allows the user to be notified of the occurrence of an abnormality.

Next, the detection of an improper immersion error will be described through reference to Fig. 13C.

If the sensing unit 31a of the sensor 30 is sufficiently immersed in the medium X, the operational amplifier output connected to the counter electrode 31ab and the operational amplifier inverting input connected to the reference electrode 31ac are connected via the liquid, forming a closed loop. The operational amplifier output voltage connected to the counter electrode 31ab varies within a specific range so that "the voltage of the operational amplifier inverting input" connected to the reference electrode 31ac and "the voltage of the operational amplifier non-inverting input" connected to the DA converter used for setting the reference electrode voltage will be equal.

On the other hand, if an immersion error occurs in which the sensing unit 31a of the sensor 30 is not sufficiently immersed in the medium X, the operational amplifier output connected to the counter electrode 31ab and the operational amplifier inverting input connected to the reference electrode 31ac are not connected via the liquid, resulting in an open loop.

Therefore, the operational amplifier operates as a comparator, and the operational amplifier output voltage (counter electrode terminal voltage) connected to the counter electrode 31ab becomes either Low or High in the initial stage (during the skip period). That is, either the counter electrode terminal voltage bottoms out (drops almost to zero) or peaks out (roughly the maximum output voltage of the operational amplifier).

If there is no problem with the immersion state at the start of voltage application, it is unlikely that an immersion error will occur midway through cell culture in a high-temperature and -humidity environment. That is, as long as the specified amount of medium X has been poured into the well 25a, there will be no immersion error even if some of the medium X evaporates during cell culture.

Consequently, the analysis unit 42 determines whether or not the counter electrode terminal voltage has deviated from the specified range during the skip period, and if it has deviated from the specified range, it is determined that an immersion error has occurred, and the display unit 41 displays that an immersion error has occurred, thereby notifying the user of the occurrence of an abnormality.

### Measurement Error Detection Method

As described above, with the cell culture analysis device 1 of this embodiment, the above-mentioned measurement errors (contamination errors and improper immersion errors) are detected by measuring the counter electrode terminal voltage related to the counter electrode 31ab of the three-electrode sensor 30.

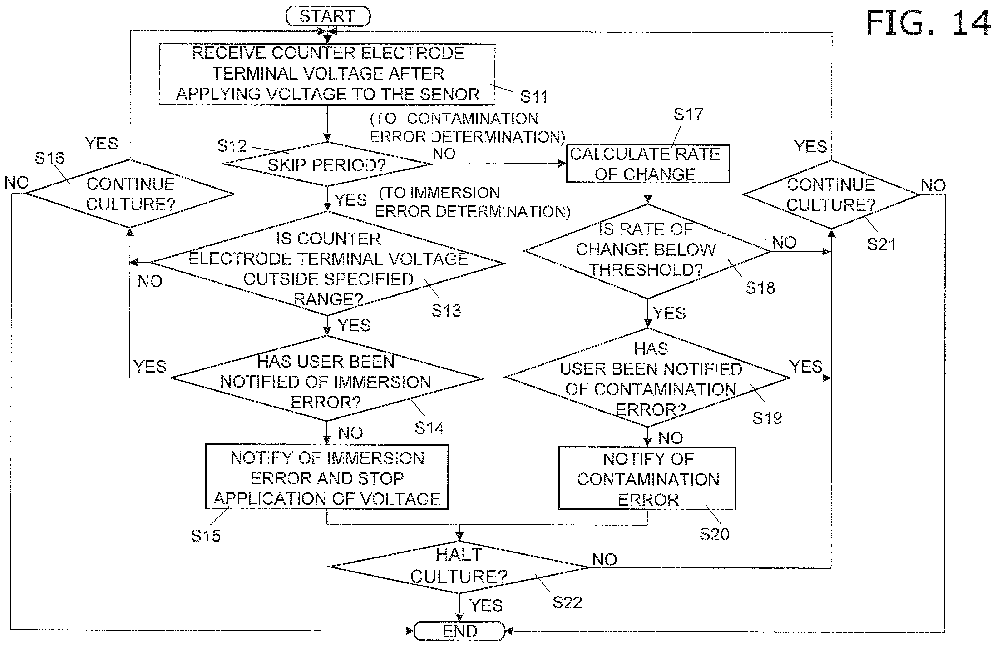

Here, the flow of processing in a measurement error detection method will be described through reference to the flowchart in Fig. 14.

That is, in step S11, after the voltage application unit 11a included in the electrochemical measurement unit 11 of the analysis unit 2 has applied a specific voltage to the sensor 30, the analysis unit 42 receives the counter electrode terminal voltage of the counter electrode 31ab included in the sensor 30, from the voltage measurement unit 11c via the communication unit 14.

Next, in step S12, the analysis unit 42 determines whether or not the received counter electrode terminal voltage is a result measured within the skip period.

Here, if the measurement result was during the skip period, the processing proceeds to the immersion error determination processing from step S13 onward. On the other hand, if the measurement result was not during the skip period, and was instead a measurement result after the skip period had elapsed, the processing proceeds to the contamination error determination processing from step S17 onward.

Next, in step S13, since the received counter electrode terminal voltage was determined in step S12 to be a measurement result during the skip period, the analysis unit 42 determines whether or not the value of the counter electrode terminal voltage is outside the specified range in order to determine whether there is an immersion error.

Here, if the value of the counter electrode terminal voltage is outside the specified range, it is determined that an immersion error has occurred, and the processing proceeds to step S14. On the other hand, if the value of the counter electrode terminal voltage is within the specified range, it is determined that no immersion error has occurred, and the processing proceeds to step S16.

Next, in step S14, since it was determined in step S13 that the value of the counter electrode terminal voltage was outside the specified range, it is determined whether or not the user has been notified on the display unit 41 about the immersion error that appears to have occurred.

Here, if notification has been given, the processing proceeds to step S16, but if no notification has been given, the processing proceeds to step S15.

Next, in step S15, in order to notify of the occurrence of an immersion error that has not yet been reported, the display unit 41 displays a message to the effect that an immersion error has occurred. If the voltage is still being applied in a state in which the sensing unit 31a of the sensor 30 is not sufficiently immersed in the culture medium X, there is the risk that the sensor 30 will malfunction, so the control unit 12 halts the application of voltage from the voltage application unit 11a to the sensor 30, and the processing proceeds to step S22.

Next, in step S16, since it was determined in step S13 that the value of the counter electrode terminal voltage was within the specified range, or it was determined in step S14 that the occurrence of an immersion error had been reported, it is determined whether or not to continue the culture.

Here, if the user selects to continue the culture, the processing goes back to step S11 and measurement error determination processing is repeated. On the other hand, if the user chooses not to continue the culture, the processing is ended.

On the other hand, in step S17, since the received counter electrode terminal voltage was determined in step S12 to be a measurement result outside the skip period, the analysis unit 42 calculates the negative rate of change in the counter electrode terminal voltage over the elapsed time in order to determine whether or not there is a contamination error.

Next, in step S18, it is determined whether or not the negative rate of change in the counter electrode terminal voltage calculated in step S17 is below a specific threshold value.

Here, if this rate of change is below the threshold value, it is determined that a contamination error has occurred, and the processing proceeds to step S19. On the other hand, if the rate of change is at or above the threshold, the processing proceeds to step S21.

Next, in step S19, since it was determined in step S18 that the negative rate of change of the counter electrode terminal voltage was below the specified threshold, it is determined whether or not the display unit 41 has notified the user that a contamination error seems to have occurred.

Here, if the user has been notified, the processing proceeds to step S21, but if the user has not been notified, the processing proceeds to step S20.

Next, in step S20, the display unit 41 displays that a contamination error has occurred in order to notify of the occurrence of a contamination error that has not yet been reported.

Next, in step S21, since it was determined in step S18 that the rate of change of the counter electrode terminal voltage was at or above the specific threshold value, or it was determined in step S19 that the user has been notified of the occurrence of a contamination error, it is determined whether or not to continue the culture. Here, if the user chooses to continue the culture, the processing returns to step S11 and measurement error determination processing is repeated. On the other hand, if the user chooses not to continue the culture, the processing is ended.

Next, in step S22, after notifying of the occurrence of an immersion error in step S15, and notifying of the occurrence of a contamination error in step S20, it is selected whether or not to halt the culture.

At this point, the user can choose whether or not to halt the culture, but normally if mold or the like has adhered, it is determined that the culture cannot be continued, and the processing is ended.

### Main Features

As shown in Fig. 10, the cell culture analysis device 1 of this embodiment comprises the voltage application unit 11a, the current measurement unit 11b, the voltage measurement unit 11c, and the analysis unit 42. The voltage application unit 11a applies voltage to the sensing unit 31a in a state in which the sensing unit 31a of the sensor 30, which includes the working electrodes 31aa and 31ad, the counter electrode 31ab, and the reference electrode 31ac, is immersed in the medium X. The current measurement unit 11b measures the current flowing through the sensor 30 based on the voltage applied to the sensing unit 31a. The analysis unit 42 calculates the concentrations of glucose and lactic acid contained in the medium X on the basis of the measurement result from the current measurement unit 11b. The voltage measurement unit 11c measures the terminal voltage of the counter electrode 31ab included in the sensing unit 31a based on the voltage applied by the voltage application unit 11a. The analysis unit 42 detects a measurement error on the basis of the terminal voltage of the counter electrode 31ab measured by the voltage measurement unit 11c.

Consequently, if the sensing unit 31a of the sensor 30 is not sufficiently immersed in the medium X, or if mold or another such impurity has adhered to the surface of the sensor 30, the occurrence of measurement errors can be detected with high accuracy by monitoring changes in the counter electrical terminal voltage indicating a certain behavior.

As a result, measurement errors caused by improper sensor immersion, contamination, or the like can be detected with high accuracy, and glucose, lactic acid, or some other such measurement target can be accurately measured.

### Other Embodiments

An embodiment of the present invention was described above, but the present invention is not limited to the above embodiment, and various changes are possible without departing from the gist of the invention.
(A) In the above embodiment, an example was given in which the present invention was realized as a sample measurement device and a sample measurement method. However, the present invention is not limited to this.

For example, the present invention may be realized as a sample measurement program that causes a computer to execute the sample measurement method featuring the sample measurement device described above.

This sample measurement program is stored in a memory (storage unit) installed in the sample measurement device, and the CPU reads the sample measurement program stored in the memory and causes the hardware to execute the various steps. More specifically, the CPU reads the sample measurement program and executes the above-mentioned voltage application step, current measurement step, concentration measurement step, counter terminal voltage measurement step, and measurement error detection step, which allows the same effect as above to be obtained.

Also, the present invention may be realized as a recording medium that stores a sample measurement program.

(B)
In the above embodiment, an example was given in which the concentration measurement unit and the measurement error detection unit included in the sample measurement device of the present invention were provided as the analysis unit 42 on the control unit 4 side. However, the present invention is not limited to this.

For example, the configuration may be such that the control unit 12 on the analysis unit 2 side functions as a concentration measurement unit and a measurement error detection unit.

(C)
In the above embodiment, an example was given in which the sensor 30 included the two working electrodes 31aa and 31ad for glucose measurement and lactic acid measurement, but the present invention is not limited to this.

For example, the sensor may have a three-electrode configuration provided with one working electrode, one counter electrode, and one reference electrode.

(D)
In the above embodiment, an example was given in which the sample measurement device of the present invention was applied to the cell culture analysis device 1. However, the present invention is not limited to this.

For example, the present invention may be applied to a device other than one used in cell culture, and to a measurement device that measures a sample.

(E)
In the above embodiment, an example was given in which the sensor 30 measured the concentrations of glucose and lactic acid as the measurement targets. However, the present invention is not limited to this.

For example, the measurement target is not limited to glucose and lactic acid, and may be some other substance.

(F)
In the above embodiment, an example was given in which the sensor 30 was used in a state of having been bent at the bent portion 32. However, the present invention is not limited to this.

For example, a sensor that has not been bent may be used.

Here again, by using a configuration in which a plurality of sensors are linked by a linking portion at the upper end portion of the main body of each sensor affords the same effect as described above, that of improving the positional accuracy of the sensors.

(G)
In the above embodiment, an example was given in which the sensor 30 was used, which was substantially an upside-down T-shape. However, the present invention is not limited to this.

For example, a substantially I-shaped or substantially L-shaped sensor may be used instead.

(H)
In the above embodiment, an example was given in which the sample measurement device of the present invention was applied to a cell culture analysis device. However, the present invention is not limited to this.

For example, the present invention may be applied to a device that measures samples unrelated to cell culture.

### INDUSTRIAL APPLICABILITY

The sample measurement device of the present invention exhibits the effect that measurement errors attributable to improper sensor immersion, contamination, etc., can be detected in order to take more accurate measurements, and is therefore not limited to the field of cell culture, and is broadly applicable to devices that measure various kinds of sample.

### REFERENCE SIGNS LIST

1 cell culture analysis device (sample measurement device)
2 analysis unit
3 incubator
3a door
4 control unit
5 electrical cable
11 electrochemical measurement unit
11a voltage application unit
11b current measurement unit
11c voltage measurement unit (counter electrode terminal voltage measurement unit)
12 control unit
13 storage unit
14 communication unit
20 sensor-equipped culture module
21 main body
22 pull-out part
23 lifting mechanism
25 well plate
25a well
27 bottom plate
28 sensor unit
30 sensor (sample measurement sensor)
31 main body
31a sensing unit (electrode unit)
31aa working electrode
31ab counter electrode
31ac reference electrode
31ad working electrode
31b connecting terminal portion
31c electrode
32 bent portion
33 linking portion
34 base material (PET sheet)
35 reagent layer
36 adhesive layer
37 protective film
38 insulating layer
41 display unit
42 analysis unit (concentration measurement unit, measurement error detection unit)
C1 biological safety cabinet
i current
X medium

## Claims

1. A sample measurement device (1), comprising:
a voltage application unit (11a) configured to apply voltage to an electrode unit (31a) including at least a working electrode (31aa, 31ad), a counter electrode (31ab), and a reference electrode (31ac) of a sample measurement sensor (30) in a state in which the electrode unit (31a) is immersed in a sample (X);
a current measurement unit (11b) configured to measure a current flowing through the sample measurement sensor (30) by the voltage applied to the electrode unit (31a);
a concentration measurement unit (42) configured to calculate a concentration of an analyte contained in the sample (X) on the basis of a measurement result from the current measurement unit (11b);
a counter electrode terminal voltage measurement unit (11c) configured to measure a terminal voltage of the counter electrode (31ab) included in the electrode unit (31a) in a state in which a voltage is being applied by the voltage application unit (11a); and
a measurement error detection unit (42) configured to detect a measurement error on the basis of the terminal voltage of the counter electrode (31ab) measured by the counter electrode terminal voltage measurement unit (11c);
the measurement error detection unit (42) is configured to detect a contamination error attributable to an admixture of an impurity other than the sample (X) adhering to a surface of the sample measurement sensor (30);
the sample measurement device (1) being **characterized in that**
the measurement error detection unit (42) is configured to determine that there is a contamination error when a fluctuation rate of the measurement result in the counter electrode terminal voltage measurement unit (11c) is at or below a specific threshold.

2. The sample measurement device (1) according to claim 1,
wherein the measurement error detection unit (42) sets a skip period in which the measurement is not performed until a specific time has elapsed since the voltage was applied from the voltage application unit (11a), and it is determined whether or not there is a contamination error after the skip period has elapsed.

3. The sample measurement device (1) according to claim 1 or 2,
wherein the measurement error detection unit (42) detects an improper immersion error caused by improper immersion in which the electrode unit (31a) is not properly immersed in the sample (X).

4. The sample measurement device (1) according to claim 3,
wherein the measurement error detection unit (42) sets a skip period in which the measurement is not performed until a specific time has elapsed since voltage was applied from the voltage application unit (11a).

5. The sample measurement device (1) according to claim 4,
wherein the measurement error detection unit (42) determines that there is an improper immersion error when the terminal voltage of the counter electrode (31ab) sensed during the skip period is outside a specific range.

6. The sample measurement device (1) according to claim 5,
wherein the voltage application unit (11a) stops an application of voltage when it is determined that there is an improper immersion error.

7. The sample measurement device (1) according to any of claims 1 to 6,
further comprising a display unit (41) configured to display a detection result for the measurement error in the measurement error detection unit (42).

8. The sample measurement device (1) according to any of claims 1 to 7,
wherein the sample measurement sensor (30) comprises the electrode unit (31a) including at least the working electrode (31aa, 31ad), the counter electrode (31ab), and the reference electrode (31ac),
in the working electrode (31aa, 31ad), a reagent applied to a surface of the electrode unit (31a) is covered with a protective film (37),
in the reference electrode (31ac), the surface of the electrode unit (31a) is covered with a protective film (37), and
in the counter electrode (31ab), an electrode portion is exposed to the sample (X).

9. The sample measurement device (1) according to claim 8,
wherein at least one of the working electrode (31aa, 31ad) and the reference electrode (31ac) is such that the surface of the electrode portion is formed in a substantially circular shape in plan view.

10. The sample measurement device (1) according to claim 8 or 9,
wherein in the counter electrode (31ab), the surface of the electrode unit (31a) is formed in a substantially rectangular shape in plan view.

11. The sample measurement device (1) according to any of claims 8 to 10,
wherein the electrode portion of the counter electrode (31ab) is formed from carbon.

12. A sample measurement method, comprising:
a voltage application step of applying voltage to an electrode unit (31a) in a state in which the electrode unit (31a) including at least a working electrode (31aa, 31ad), a counter electrode (31ab), and a reference electrode (31ac) of a sample measurement sensor (30) is immersed in a sample (X);
a current measurement step of measuring a current flowing through the sample measurement sensor (30) by the voltage applied in the voltage application step;
a concentration measurement step of calculating a concentration of an analyte contained in the sample (X) on the basis of a measurement result in the current measurement step;
a counter electrode terminal voltage measurement step of measuring a terminal voltage of the counter electrode (31ab) included in the electrode unit (31a) in a state in which voltage is being applied in the voltage applying step; and
a measurement error detection step of detecting a measurement error on the basis of the terminal voltage of the counter electrode (31ab) measured in the counter electrode terminal voltage measurement step;
in the measurement error detection step, a contamination error attributable to an admixture of an impurity other than the sample (X) adhering to a surface of the sample measurement sensor (30) is detected;
the sample measurement method being **characterized in that**
in the measurement error detection step, it is determined that there is a contamination error when a fluctuation rate of the measurement result in the counter electrode terminal voltage measurement step is at or below a specific threshold.

13. A sample measurement program comprising instructions to cause the sample measurement device of any of claims 1 to 11 to execute a sample measurement method comprising:
a voltage application step of applying voltage to an electrode unit (31a) in a state in which the electrode unit (31a) including at least a working electrode (31aa, 31ad), a counter electrode (31ab), and a reference electrode (31ac) of a sample measurement sensor (30) is immersed in a sample (X);
a current measurement step of measuring a current flowing through the sample measurement sensor (30) by the voltage applied in the voltage application step;
a concentration measurement step of calculating a concentration of an analyte contained in the sample (X) on the basis of a measurement result in the current measurement step;
a counter electrode terminal voltage measurement step of measuring a terminal voltage of the counter electrode (31ab) included in the electrode unit (31a) in a state in which voltage is being applied in the voltage applying step; and
a measurement error detection step of detecting a measurement error on the basis of the terminal voltage of the counter electrode (31ab) measured in the counter electrode terminal voltage measurement step;
in the measurement error detection step, a contamination error attributable to an admixture of an impurity other than the sample (X) adhering to a surface of the sample measurement sensor (30) is detected;
the sample measurement program being **characterized in that**
in the measurement error detection step, it is determined that there is a contamination error when a fluctuation rate of the measurement result in the counter electrode terminal voltage measurement step is at or below a specific threshold.

## Patentansprüche

1. Probenmessungs-Vorrichtung (1), die umfasst:
eine Spannungsanlege-Einheit (11a), die so ausgeführt ist, dass sie Spannung an eine Elektroden-Einheit (31a), die wenigstens eine Arbeitselektrode (31aa, 31ad), eine Gegenelektrode (31ab) sowie eine Bezugselektrode (31ac) eines Probenmessungs-Sensors (30) einschließt, in einem Zustand anlegt, in dem die Elektroden-Einheit (31a) in eine Probe (X) eingetaucht ist;
eine Strommessungs-Einheit (11b), die so ausgeführt ist, dass sie einen Strom misst, der aufgrund der an die Elektroden-Einheit (31a) angelegten Spannung durch den Probenmessungs-Sensor (30) fließt;
eine Konzentrationsmessungs-Einheit (42), die so ausgeführt ist, dass sie eine Konzentration eines in der Probe (X) enthaltenen Analyts auf Basis eines Messergebnisses von der Strommessungs-Einheit (11b) berechnet;
eine Einheit (11c) für Messung einer Gegenelektroden-Anschlussspannung, die so ausgeführt ist, dass sie eine Anschlussspannung der in der Elektroden-Einheit (31a) eingeschlossenen Gegenelektrode (31ab) in einem Zustand misst, in dem eine Spannung durch die Spannungsanlege-Einheit (11a) angelegt wird; sowie
eine Einheit (42) für Erfassung eines Messfehlers, die so ausgeführt ist, dass sie einen Messfehler auf Basis der durch die Einheit (11c) für Messung einer Gegenelektroden-Anschlussspannung gemessenen Anschlussspannung der Gegenelektrode (31ab) erfasst;
wobei die Einheit (42) für Erfassung eines Messfehlers so ausgeführt ist, dass sie einen Kontaminations-Fehler erfasst, der auf eine Beimischung einer von der Probe (X) verschiedenen Verunreinigung zurückzuführen ist, die an einer Oberfläche des Probenmessungs-Sensors (30) haftet;
die Probenmessungs-Vorrichtung (1) **dadurch gekennzeichnet ist, dass**
die Einheit (42) für Erfassung eines Messfehlers so ausgeführt ist, dass sie feststellt, dass ein Kontaminations-Fehler vorliegt, wenn eine Fluktuationsrate des Messergebnisses in der Einheit (11c) für Messung einer Gegenelektroden-Anschlussspannung auf oder unter einem vorgegebenen Schwellenwert liegt.

2. Probenmessungs-Vorrichtung (1) nach Anspruch 1,
wobei die Einheit (42) für Erfassung eines Messfehlers eine Aussetz-Periode festlegt, in der die Messung erst dann durchgeführt wird, wenn eine vorgegebene Zeit seit Anlegen der Spannung von der Spannungsanlege-Einheit (11a) verstrichen ist, und festgestellt wird, ob ein Kontaminations-Fehler vorliegt, nachdem die Aussetz-Periode verstrichen ist.

3. Probenmessungs-Vorrichtung (1) nach Anspruch 1 oder 2,
wobei die Einheit (42) für Erfassung eines Messfehlers einen Fehler aufgrund von falschem Eintauchen erfasst, der durch falsches Eintauchen verursacht wird, bei dem die Elektroden-Einheit (31a) nicht richtig in die Probe (X) eingetaucht wird.

4. Probenmessungs-Vorrichtung (1) nach Anspruch 3,
wobei die Einheit (42) für Erfassung eines Messfehlers eine Aussetz-Periode festlegt, in der die Messung erst dann durchgeführt wird, wenn eine vorgegebene Zeit seit Anlegen der Spannung von der Spannungsanlege-Einheit (11a) verstrichen ist.

5. Probenmessungs-Vorrichtung (1) nach Anspruch 4,
wobei die Einheit (42) für Erfassung eines Messfehlers feststellt, dass ein Fehler aufgrund von falschem Eintauchen vorliegt, wenn die während der Aussetz-Periode gemessene Anschlussspannung der Gegenelektrode (31ab) außerhalb eines vorgegebenen Bereiches liegt.

6. Probenmessungs-Vorrichtung (1) nach Anspruch 5,
wobei die Spannungsanlege-Einheit (11a) ein Anlegen von Spannung unterbricht, wenn festgestellt wird, dass ein Fehler aufgrund von falschem Eintauchen vorliegt.

7. Probenmessungs-Vorrichtung (1) nach einem der Ansprüche 1 bis 6,
die des Weiteren eine Anzeige-Einheit (41) umfasst, die so ausgeführt ist, dass sie ein Erfassungsergebnis für den Messfehler in der Einheit (42) für Erfassung eines Messfehlers anzeigt.

8. Probenmessungs-Vorrichtung (1) nach einem der Ansprüche 1 bis 7,
wobei der Probenmessungs-Sensor (30) die Elektroden-Einheit (31a) umfasst, die wenigstens die Arbeitselektrode (31aa, 31ad), die Gegenelektrode (31ab) und die Bezugselektrode (31ac) einschließt,
bei der Arbeitselektrode (31aa, 31ad) ein auf eine Oberfläche der Elektroden-Einheit (31a) aufgebrachtes Reagens mit einem Schutzfilm (37) abgedeckt ist,
bei der Bezugselektrode (31ac) die Oberfläche der Elektroden-Einheit (31a) mit einem Schutzfilm (37) abgedeckt ist, und
bei der Gegenelektrode (31ab) ein Elektrodenabschnitt zu der Probe (X) freiliegt.

9. Probenmessungs-Vorrichtung (1) nach Anspruch 8,
wobei die Arbeitselektrode (31aa, 31ad) oder/und die Bezugselektrode (31ac) so eingerichtet ist, dass die Oberfläche des Elektrodenabschnitts in Draufsicht im Wesentlichen kreisförmig ausgebildet ist.

10. Probenmessungs-Vorrichtung (1) nach Anspruch 8 oder 9,
wobei bei der Gegenelektrode (31ab) die Oberfläche der Elektroden-Einheit (31a) in Draufsicht in einer im Wesentlichen rechteckigen Form ausgebildet ist.

11. Probenmessungs-Vorrichtung (1) nach einem der Ansprüche 8 bis 10,
wobei der Elektrodenabschnitt der Gegenelektrode (31ab) aus Kohlenstoff besteht.

12. Probenmessungs-Verfahren, das umfasst:
einen Spannungsanlege-Schritt, in dem Spannung an eine Elektroden-Einheit (31a) in einem Zustand angelegt wird, in dem die Elektroden-Einheit (31a), die wenigstens eine Arbeitselektrode (31aa, 31ad), eine Gegenelektrode (31ab) sowie eine Bezugselektrode (31ac) eines Probenmessungs-Sensors (30) einschließt, in eine Probe (X) eingetaucht ist; einen Strommessungs-Schritt, in dem ein Strom gemessen wird, der aufgrund der in dem Spannungsanlege-Schritt angelegten Spannung durch den Probenmessungs-Sensor (30) fließt;
einen Konzentrationsmessungs-Schritt, in dem eine Konzentration eines in der Probe (X) enthaltenen Analyts auf Basis eines Messergebnisses in dem Strommessungs-Schritt berechnet wird;
einen Schritt für Messung einer Gegenelektroden-Anschlussspannung, in dem eine Anschlussspannung der in der Elektroden-Einheit (31a) eingeschlossenen Gegenelektrode (31ab) in einem Zustand gemessen wird, in dem in dem Spannungsanlege-Schritt Spannung angelegt wird; sowie
einen Schritt für Erfassung eines Messfehlers, in dem ein Messfehler auf Basis der in dem Schritt für Messung einer Gegenelektroden-Anschlussspannung gemessenen Anschlussspannung der Gegenelektrode (31ab) erfasst wird;
wobei in dem Schritt für Erfassung eines Messfehlers ein Kontaminations-Fehler erfasst wird, der auf eine Beimischung einer von der Probe (X) verschiedenen Verunreinigung zurückzuführen ist, die an einer Oberfläche des Probenmessungs-Sensors (30) haftet;
und das Probenmessungs-Verfahren **dadurch gekennzeichnet ist, dass**
in dem Schritt für Erfassung eines Messfehlers festgestellt wird, dass ein Kontaminations-Fehler vorliegt, wenn eine Fluktuationsrate des Messergebnisses in dem Schritt für Messung einer Gegenelektroden-Anschlussspannung auf oder unter einem vorgegebenen Schwellenwert liegt.

13. Probenmessungs-Programm, das Befehle umfasst, die die Probenmessungs-Vorrichtung nach einem der Ansprüche 1 bis 11 veranlassen, ein Probenmessungs-Verfahren auszuführen, das umfasst:
einen Spannungsanlege-Schritt, in dem Spannung an eine Elektroden-Einheit (31a) in einem Zustand angelegt wird, in dem die Elektroden-Einheit (31a), die wenigstens eine Arbeitselektrode (31aa, 31ad), eine Gegenelektrode (31ab) sowie eine Bezugselektrode (31ac) eines Probenmessungs-Sensors (30) einschließt, in eine Probe (X) eingetaucht ist; einen Strommessungs-Schritt, in dem ein Strom gemessen wird, der aufgrund der in dem Spannungsanlege-Schritt angelegten Spannung durch den Probenmessungs-Sensor (30) fließt;
einen Konzentrationsmessungs-Schritt, in dem eine Konzentration eines in der Probe (X) enthaltenen Analyts auf Basis eines Messergebnisses in dem Strommessungs-Schritt berechnet wird;
einen Schritt für Messung einer Gegenelektroden-Anschlussspannung, in dem eine Anschlussspannung der in der Elektroden-Einheit (31a) eingeschlossenen Gegenelektrode (31ab) in einem Zustand gemessen wird, in dem in dem Spannungsanlege-Schritt Spannung angelegt wird; sowie
einen Schritt für Erfassung eines Messfehlers, in dem ein Messfehler auf Basis der in dem Schritt für Messung einer Gegenelektroden-Anschlussspannung gemessenen Anschlussspannung der Gegenelektrode (31ab) erfasst wird;
wobei in dem Schritt für Erfassung eines Messfehlers ein Kontaminations-Fehler erfasst wird, der auf eine Beimischung einer von der Probe (X) verschiedenen Verunreinigung zurückzuführen ist, die an einer Oberfläche des Probenmessungs-Sensors (30) haftet;
und das Probenmessungs-Programm **dadurch gekennzeichnet ist, dass**
in dem Schritt für Erfassung eines Messfehlers festgestellt wird, dass ein Kontaminations-Fehler vorliegt, wenn eine Fluktuationsrate des Messergebnisses in dem Schritt für Messung einer Gegenelektroden-Anschlussspannung auf oder unter einem vorgegebenen Schwellenwert liegt.

## Revendications

1. Dispositif de mesure d'échantillon (1), comprenant :
une unité d'application de tension (11a) configurée pour appliquer une tension à une unité électrode (31a) incluant au moins une électrode de travail (31aa, 31ad), une contre-électrode (31ab), et une électrode de référence (31ac) d'un capteur de mesure d'échantillon (30) sous un état dans lequel l'unité électrode (31a) est immergée dans un échantillon (X) ;
une unité de mesure de courant (11b) configurée pour mesurer un courant s'écoulant à travers le capteur de mesure d'échantillon (30) par la tension appliquée à l'unité électrode (31a) ;
une unité de mesure de concentration (42) configurée pour calculer une concentration d'un analyte contenu dans l'échantillon (X) sur la base d'un résultat de mesure à partir de l'unité de mesure de courant (11b) ;
une unité de mesure de tension terminale de contre-électrode (11c) configurée pour mesurer une tension terminale de la contre-électrode (31ab) incluse dans l'unité électrode (31a) sous un état dans lequel une tension est appliquée par l'unité d'application de tension (11a) ; et
une unité de détection d'erreur de mesure (42) configurée pour détecter une erreur de mesure sur la base de la tension terminale de la contre-électrode (31ab) mesurée par l'unité de mesure de tension terminale de contre-électrode (11c) ;
l'unité de détection d'erreur de mesure (42) est configurée pour détecter une erreur de contamination pouvant être attribuée à un mélange par admixtion d'une impureté autre que l'échantillon (X) adhérant à une surface du capteur de mesure d'échantillon (30) ;
le dispositif de mesure d'échantillon (1) étant **caractérisé en ce que**
l'unité de détection d'erreur de mesure (42) est configurée pour déterminer qu'il existe une erreur de contamination lorsqu'un taux de fluctuation du résultat de mesure dans l'unité de mesure de tension terminale de contre-électrode (11c) se trouve au niveau, ou en-dessous, d'un seuil spécifique.

2. Dispositif de mesure d'échantillon (1) selon la revendication 1,
dans lequel l'unité de détection d'erreur de mesure (42) définit une période de saut dans laquelle la mesure n'est pas effectuée jusqu'à ce qu'une durée spécifique se soit écoulée depuis que la tension a été appliquée depuis l'unité d'application de tension (11a), et il est déterminé ou pas qu'il existe une erreur de contamination après que la période de saut s'est écoulée.

3. Dispositif de mesure d'échantillon (1) selon la revendication 1 ou 2,
dans lequel l'unité de détection d'erreur de mesure (42) détecte une erreur d'immersion incorrecte causée par une immersion incorrecte dans laquelle l'unité électrode (31a) n'est pas correctement immergée dans l'échantillon (X).

4. Dispositif de mesure d'échantillon (1) selon la revendication 3,
dans lequel l'unité de détection d'erreur de mesure (42) définit une période de saut dans laquelle la mesure n'est pas effectuée jusqu'à ce qu'une durée spécifique s'est écoulée depuis que la tension a été appliquée depuis l'unité d'application de tension (11a).

5. Dispositif de mesure d'échantillon (1) selon la revendication 4,
dans lequel l'unité de détection d'erreur de mesure (42) détermine qu'il existe une erreur d'immersion incorrecte lorsque la tension terminale de la contre-électrode (31ab) détectée durant la période de saut se trouve à l'extérieur d'une plage spécifique.

6. Dispositif de mesure d'échantillon (1) selon la revendication 5,
dans lequel l'unité d'application de tension (11a) arrête une application de tension lorsqu'il est déterminé qu'il existe une erreur d'immersion incorrecte.

7. Dispositif de mesure d'échantillon (1) selon l'une quelconque des revendications 1 à 6,
comprenant en outre une unité d'affichage (41) configurée pour afficher un résultat de détection pour l'erreur de mesure dans l'unité de détection d'erreur de mesure (42).

8. Dispositif de mesure d'échantillon (1) selon l'une quelconque des revendications 1 à 7,
dans lequel le capteur de mesure d'échantillon (30) comprend l'unité électrode (31a) incluant au moins l'électrode de travail (31aa, 31ad), la contre-électrode (31ab), et l'électrode de référence (31ac),
dans l'électrode de travail (31aa, 31ad), un réactif appliqué à une surface de l'unité électrode (31a) est recouvert d'un film protecteur (37),
dans l'électrode de référence (31ac), la surface de l'unité électrode (31a) est recouverte d'un film protecteur (37), et
dans la contre-électrode (31ab), une portion d'électrode est exposée à l'échantillon (X).

9. Dispositif de mesure d'échantillon (1) selon la revendication 8,
dans lequel au moins l'une de l'électrode de travail (31aa, 31ad) et de l'électrode de référence (31ac) est telle que la surface de la portion d'électrode est formée en une forme sensiblement circulaire en vue en plan.

10. Dispositif de mesure d'échantillon (1) selon la revendication 8 ou 9,
dans lequel dans la contre-électrode (31ab), la surface de l'unité électrode (31a) est formée en une forme sensiblement rectangulaire en vue en plan.

11. Dispositif de mesure d'échantillon (1) selon l'une quelconque des revendications 8 à 10,
dans lequel la portion d'électrode de la contre-électrode (31ab) est formée à partir de carbone.

12. Procédé de mesure d'échantillon, comprenant :
une étape d'application de tension d'application d'une tension à une unité électrode (31a) sous un état dans lequel l'unité électrode (31a) incluant au moins une électrode de travail (31aa, 31ad), une contre-électrode (31ab), et une électrode de référence (31ac) d'un capteur de mesure d'échantillon (30) est immergée dans un échantillon (X) ;
une étape de mesure de courant afin de mesurer un courant s'écoulant à travers le capteur de mesure d'échantillon (30) par la tension appliquée dans l'étape d'application de tension ;
une étape de mesure de concentration afin de calculer une concentration d'un analyte contenu dans l'échantillon (X) sur la base d'un résultat de mesure dans l'étape de mesure de courant ;
une étape de mesure de tension terminale de contre-électrode afin de mesurer une tension terminale de la contre-électrode (31ab) incluse dans l'unité électrode (31a) sous un état dans lequel une tension est appliquée dans l'étape d'application de tension ; et
une étape de détection d'erreur de mesure afin de détecter une erreur de mesure sur la base de la tension terminale de la contre-électrode (31ab) mesurée dans l'étape de mesure de la tension terminale de la contre-électrode ;
dans l'étape de détection d'erreur de mesure, une erreur de contamination pouvant être attribuée à un mélange par admixtion d'une impureté autre que l'échantillon (X) adhérant à une surface du capteur de mesure d'échantillon (30) est détectée ;
le procédé de mesure d'échantillon étant **caractérisé en ce que**
dans l'étape de détection d'erreur de mesure, il est déterminé qu'il existe une erreur de contamination lorsqu'un taux de fluctuation du résultat de mesure dans l'étape de mesure de la tension terminale de la contre-électrode se trouve au niveau, ou en-dessous, d'un seuil spécifique.

13. Programme de mesure d'échantillon comprenant des instructions pour amener le dispositif de mesure d'échantillon selon l'une quelconque des revendications 1 à 11 à exécuter un procédé de mesure d'échantillon comprenant :
une étape d'application de tension afin d'appliquer une tension à une unité électrode (31a) sous un état dans lequel l'unité électrode (31a) incluant au moins une électrode de travail (31aa, 31ad), une contre-électrode (31ab), et une électrode de référence (31ac) d'un capteur de mesure d'échantillon (30) est immergée dans un échantillon (X) ;
une étape de mesure de courant afin de mesurer un courant s'écoulant à travers le capteur de mesure d'échantillon (30) par la tension appliquée dans l'étape d'application de tension ;
une étape de mesure de concentration afin de calculer une concentration d'un analyte contenu dans l'échantillon (X) sur la base d'un résultat de mesure dans l'étape de mesure de courant ;
une étape de mesure de tension terminale de contre-électrode afin de mesurer une tension terminale de la contre-électrode (31ab) incluse dans l'unité électrode (31a) sous un état dans lequel une tension est appliquée dans l'étape d'application de tension ; et
une étape de détection d'erreur de mesure afin de détecter une erreur de mesure sur la base de la tension terminale de la contre-électrode (31ab) mesurée dans l'étape de mesure de la tension terminale de la contre-électrode ;
dans l'étape de détection d'erreur de mesure, une erreur de contamination pouvant être attribuée à un mélange par admixtion d'une impureté autre que l'échantillon (X) adhérant à une surface du capteur de mesure d'échantillon (30) est détectée ;
le programme de mesure d'échantillon étant **caractérisé en ce que**
dans l'étape de détection d'erreur de mesure, il est déterminé qu'il existe une erreur de contamination lorsqu'un taux de fluctuation du résultat de mesure dans l'étape de mesure de la tension terminale de la contre-électrode se trouve au niveau, ou en-dessous, d'un seuil spécifique.
